# EUROPÄISCHE PATENTSCHRIFT

(11) **EP 0 652 784 B1**
(45) Veröffentlichungstag und Bekanntmachung des Hinweises auf die Patenterteilung: **17.03.1999**
(21) Anmeldenummer: 93900033.7
(22) Anmeldetag: 11.12.1992
(51) Int. Cl.: A61M 15/00, B05B 11/06, B05B 11/00

(54) **PULVERINHALATOR MIT PULVERTRÄGER AUS REGELMÄSSIGEN MIKROSTRUKTUREN**
POWDER INHALER USING POWDER CARRIERS WITH REGULAR SURFACE MICROFEATURES
INHALATEUR A POUDRE MUNI DE SUPPORTS DE POUDRE A MICROSTRUCTURES REGULIERES

(30) Priorität: 20.12.1991 DE 4142238
(43) Veröffentlichungstag der Anmeldung: 17.05.1995
(73) Patentinhaber: BOEHRINGER INGELHEIM INTERNATIONAL GmbH, 55218 Ingelheim am Rhein (DE); Boehringer Ingelheim Pharma KG, 55216 Ingelheim am Rhein (DE)
(72) Erfinder: ZIERENBERG, Bernd, D-6530 Bingen (DE); HOCHRAINER, Dieter, D-6530 Bingen (DE)
(74) Vertreter: Laudien, Dieter, Dr.
(86) Internationale Anmeldenummer: EP9202867
(87) Internationale Veröffentlichungsnummer: WO9312831

(56) Entgegenhaltungen:
- WO-A-90/13328
- WO-A-92/00115
- DE-A- 4 015 367
- US-A- 3 856 185

## Beschreibung

Die Erfindung betrifft Pulverinhalatoren gemäß dem Oberbegriff des Anspruchs 1, in denen das für die Inhalation verwendete Pulver auf Trägern mit bestimmten regelmäßigen Mikrostrukturen bereitgestellt wird.

Unter den zahlreichen Gerätetypen für die Pulverinhalation, die bereits beschrieben wurden, finden sich auch solche mit bandförmigen Trägern (siehe WO 90/13328, die zur Bildung des Obergriffs des Anspruchs 1 herangezogen worden ist.). die zur Bildung des Oberbegriffs des Anspruch 1 herangezogen worden ist. Solche Träger können sehr verschiedene Oberflächenstrukturen, z.B. in Kunststoffolie eingeprägte kegelförmige Vertiefungen zur Aufnahme des Pulvers, aufweisen oder beispielsweise aus gewebtem oder nichtgewebtem (vliesartigem) Fasermaterial bestehen, bei dem das Pulver zwischen die Fasern oder in Lücken des samt- oder veloursartigen Gewebes eingelagert sind (WO 92/00115, veröffentlicht nach dem Prioritätstag der vorliegenden Erfindung).

Wie nun gefunden wurde, sind auf der Trägerfläche regelmäßig angeordnete Mikrostrukturen für die Speicherung und Ausbringung des Inhalationspulvers besonders günstig, wenn sie gemäß dem Kennzeichen des Anspruchs 1 ausgebildet sind.

Die Mikrostrukturen sind einzelne noppenartige Erhebungen auf dem ebenen, band- oder platten- bzw. scheibenförmigen Träger. Die Mikrostrukturen können die verschiedenartigsten Formen haben, z.B. zylindrisch, prismatisch, kegel- bzw. kegelstumpfförmig oder pyramiden- bzw. pyramidenstumpfförmig sein. Die auf den Träger gerichtete Achse ist bevorzugt senkrecht auf diesen ausgerichtet, kann mit ihm aber auch einen Winkel <90° bilden. Der Querschnitt der Mikrostrukturen kann entsprechend dem oben Gesagten gleichbleibend sein, kann sich aber auch mit zunehmendem Abstand von dem Träger in Form und Fläche ändern. Der Querschnitt hat bevorzugt Kreis- oder Ellipsenform oder stellt ein regelmäßiges Vieleck dar. Er kann aber auch eine unregelmäßige Form haben, etwa ein unregelmäßiges Vieleck oder eine Kombination von runden und eckigen Formen darstellen, Y- oder kreuzförmig oder linsenförmig sein.

Die Höhe der Mikrostrukturen liegt zwischen etwa 10 und 500 µm, bevorzugt ist der Bereich von 50 bis 200 µm. Der Durchmesser bzw. die entsprechenden Seitenabstände bei nicht-kreisförmigem Querschnitt beträgt zwischen etwa 10 und 500 µm, bevorzugt 30 bis 100 µm.

Für den Abstand zwischen den Mikrostrukturen haben sich etwa 10 bis 500 µm, vor allem etwa 30 bis 100 µm als günstig erwiesen.

Die Oberflächen sind in der Regel sehr glatt (optisch spiegelnd) und die Rauhtiefe beträgt ca. 50 nm. Möglich ist für den vorgesehenen Zweck jedoch auch eine deutlich größere Rauhigkeit. Beispielsweise liegt bei Mikrostrukturen, die mit Hilfe von Ätzverfahren hergestellt werden, die Rauhtiefe in der Größenordnung von 10 µm.

Die Porosität ist das Verhältnis von freiem Volumen zwischen den Mikrostrukturen zum gesamten Raum, der mit Mikrostrukturen ausgefüllt ist. Im Falle von prismatischen oder zylindrischen Strukturen ist dies gleich dem Verhältnis der nicht von Mikrostrukturen bedeckten Fläche zur Gesamtfläche.

Die Porosität kann durch die Auswahl der Formen, Größen und Abstände der Mikrostrukturen in weiten Grenzen eingestellt werden, z.B. im Bereich von 10 bis 98%. Typische Werte liegen im Bereich von 50 bis 80 %. Die Wahl des Wertes hängt auch von der Art und Dosisgröße des verwendeten Arzneistoffs ab. In der Regel ist bei besonders kleinen Dosen ein Träger zu bevorzugen, der eine höhere Dichte der Mikrostrukturen, d.h. eine geringere Porosität aufweist.

Die erfindungsgemäß verwendeten Träger werden bevorzugt in Spritzgußtechnik hergestellt. Dementsprechend werden in der Regel Materialien verwendet, die in dieser Technik verarbeitet werden können, etwa Polymethacrylat, Polyamid, Polyoximethylen.

Die Beladung der Mikrostrukturen mit Pulver kann z.B. so erfolgen, daß Pulver auf die Mikrostrukturen aufgetragen wird und mit einer Rakel sowohl das Pulver in die Mikrostrukturen eingedrückt als auch der Überschuß abgestreift wird. Eine andere Möglichkeit besteht darin, das Pulver mit einem Pinsel oder einer Bürste in die Mikrostrukturen einzustreichen. Besonders bewährt hat sich, die Mikrostrukturen in das Pulver zu drücken, so daß das Pulver in die Zwischenräume eindringt und eine dann an der Oberfläche anhaftende, leicht verfestigte Schicht mit einer Klinge abgetragen wird. Die Haftung des Pulvers zwischen den Mikrostrukturen ist so gut, daß das Pulver selbst bei Beschleunigungskräften von einigen zehntausend Meter/Sekunde² (= einige tausend g) nicht aus den Mikrostrukturen herausgeschleudert wird.

Die Beladung pro Flächeneinheit ergibt sich im wesentlichen aus der Porosität, der Höhe der Mikrostrukturen und der Dichte des aufgebrachten Pulvers. Bei einem üblichen Broncholytikum wie Fenoterol ist die Dichte ca. 600 kg/m³. Die Beladung beträgt etwa 0,3 bis 10 mg/cm², typische Werte liegen zwischen 4 und 6 mg/cm².

Die Dosierung ergibt sich aus der Beladung und der abgeblasenen Fläche, Wobei die abgeblasene Fläche zwischen etwa 4 und 50 mm² liegen kann. Daraus ergeben sich Dosierungen zwischen etwa 0,012 und 5 mg. Typische Werte liegen zwischen 0,05 und 0,5 mg. Bei anderen Gestaltungen der Inhalationsgeräte können auch erheblich größere Flächen abgeblasen werden, jedoch ist es im allgemeinen nicht zweckmäßig, wesentlich mehr als 100 mm² pro Einzeldosis zu verwenden.

Es gibt Träger mit strukturierter Oberfläche für pulverförmige Arzneistoffe, bei denen die Strukturen aus kleinen Vertiefungen (Mulden) bestehen (z.B. gemäß EP-A-0455 463). Die Vertiefungen werden mit dem Arzneistoff gefüllt. Die Vertiefungen sind durch Stege, die die Wände der Vertiefungen bilden, getrennt. Demgegenüber ist der Raum, in dem sich erfindungsgemäß bei dieser Anmeldung die säulenförmigen Mikrostrukturen befinden, zusammenhängend. Dies hat zur Folge, daß sich beim Ausblasen des Pulvers aus diesen Mikrostrukturen eine Angriffsfläche für den Luftstrom bildet. Daraus resultiert, daß
(I) das Pulver fast ohne Rückstand aus den säulenförmigen Mikrostrukturen ausgeblasen werden kann (dagegen verbleibt z.B. bei muldenförmigen Strukturen von 45 µm Tiefe ein Rückstand von 65 % des Medikamentes in den Mulden) und
(II) andererseits die Dispergierung wesentlich besser ist als bei Oberflächen, die mit Mulden strukturiert sind. So wurde bei der Verwendung von mikronisiertem Fenoterol mit einem Luftstoß von 5 cm³ und 1 bar Überdruck ein lungengängiger Anteil in dem erzeugten Aerosol (Teilchengröße < 6 µm) von > 50 % erreicht, während der lungengängige Anteil bei einem Aerosol aus mit Mulden strukturierten Oberflächen nur bei 5 bis 15 % liegt.

Der nicht-lungengängige Anteil eines Aerosols wird überwiegend auf den Wänden des Mund- und Rachenraumes deponiert und dort resorbiert oder abgeschluckt. Der gegenüber anderen Pulververneblern erheblich größere inhalierfähige Anteil ist besonders vorteilhaft für Medikamente mit systemischen Nebenwirkungen. Diese sind bei gleicher therapeutischer Dosis umso geringer, je weniger außerhalb des Zielorganes in den Körper gelangt.

Bandförmige Träger gemäß der Erfindung können in Inhalationsgeräten verwendet werden, wie sie aus dem Stand der Technik, z.B. aus der oben erwähnten Anmeldung WO 90/13328 bekannt sind. Dabei können die neuen Pulverträger auch in an sich bekannter Weise kaschiert sein, beispielsweise mit einer Aluminiumfolie, die erst unmittelbar vor der Anwendung entfernt wird.

Als besonders praxistauglich haben sich weitgehend starre bzw. steife Träger in Form länglicher Streifen oder vor allem in Form von Kreisscheiben erwiesen.

Solche Träger lassen sich mit der sog. LIGA-Technologie herstellen [E.W. Becker, W. Ehrenfeld, P. Hagemann, A. Maner, D. Münchmeyer: Herstellung von Mikrostrukturen mit großem Aspektverhältnis und großer Strukturhöhe durch Röntgentiefenlithografie mit Synchrotronstrahlung, Galvanoformung und Kunststoffabformung (LIGA-Verfahren), Microelectonic Engineering 4 (1986), 35-56]. Bei diesem Verfahren wird zunächst eine Maske mit der gewünschten Struktur, die für Synchrotronstrahlung undurchlässig ist, hergestellt. Durch diese Maske wird ein auf Synchrotronstrahlung empfindlicher Resist bestrahlt. Im folgenden Entwicklungsschritt werden die bestrahlten Teile aus dem Resist herausgelöst und in der anschließenden Galvanoformung mit Metall ausgefüllt. Dann wird der verbleibende Resist aus der Metallstruktur herausgelöst. Diese Metallstruktur wird schließlich als Form eines speziellen Spritzgußverfahrens verwendet.

Es werden auch gute Ergebnisse mit Mikrostrukturen erzielt, die in konventioneller Spritzgußtechnik hergestellt sind. Bevorzugt sind dabei Platten oder Bänder, die mit kalottenförmigen (noppenartigen) Erhebungen versehen sind. Die Form für den Spritzguß kann in diesen Fällen durch Ätzen eines Stahlwerkzeugs hergestellt werden, wobei das Muster der Strukturen durch Projektion eines entsprechenden Musters auf einen Fotoresist vorgegeben wird.

Neben den geometrischen Eigenschaften der Mikrostrukturen spielen auch chemisch-physikalische Materialeigenschaften im Rahmen der Erfindung eine Rolle. Bei dem Wirkstoff Fenoterolhydrobromid z.B. hat sich ein Polypropylen (Novolen^{R}) als besonders günstig für die Adhäsion erwiesen. Jedoch können allgemein Kunststoffe der verschiedensten Art verwendet werden, etwa Polyamide (z.B. Vestamid^{R}, Zytel^{R}), Polycarbonate (z.B. Makrolon^{R}), Acrylnitril/Butadien/Styrol-Polymer (Terluran^{R}). Gewünschtenfalls kann der Fachmann die für seinen Zweck geeignetsten Mikrostrukturen und Materialeigenschaften durch einfache Versuche ermitteln, etwa wenn es darum geht, eine besonders gute Haftung des Pulvers am Träger zu erreichen oder umgekehrt die Freisetzung zu erleichtern.

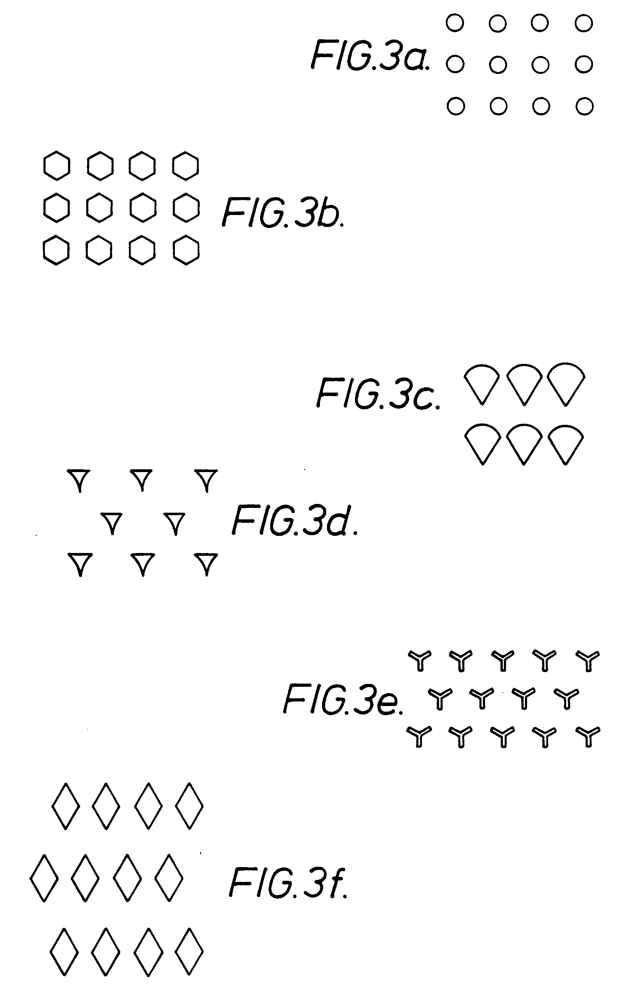

Ein Pulverinhalator, in dem Kreisscheiben als Träger verwendet werden, ist in Fig. 1 dargestellt. Die Fig. 2a bis 2b zeigen dazu passende Träger. In den Fig. 3a bis 3f sind Beispiele für die erfindungsgemäßen Mikrostrukturen dargestellt.

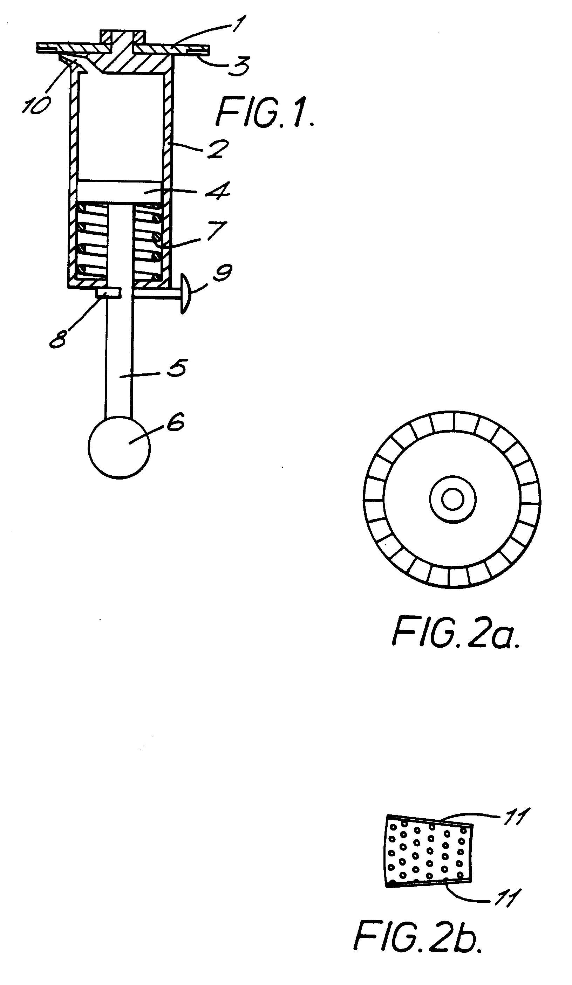

In der schematischen Darstellung eines erfindungsgemäßen Inhalators in Fig. 1 befindet sich die Trägerscheibe 1 drehbar gelagert auf dem zylindrischen Pumpenkörper 2. Die Mikrostrukturen 3 sind auf der Unterseite der Trägerscheibe ringförmig angeordnet. Die Pumpe besitzt einen Kolben 4 mit einer nach außen geführten Zugstange 5, die in einem Knopf 6 endet. Mittels der Zugstange kann der Kolben gegen den Druck einer Spiralfeder 7 nach unten gezogen und mit einem Schieber 8 im gespannten Zustand fixiert werden. Um das Gerät zur Inhalation zu benutzen, wird der Schieber 8 durch Betätigen des Druckknopfs 9 gelöst, so daß die Feder 7 den Kolben 4 nach oben drückt. Dadurch wird ein Luftstoß erzeugt, der durch die Düse 10 auf das zwischen den Mikrostrukturen eingelagerte Pulver gelenkt wird.

Die vorstehend beschriebene Vorrichtung zur Freisetzung einer Wirkstoffdosis wird zweckmäßig in ein Gehäuse eingebaut, das mit einem Mundrohr und einem Lufteinlaß versehen ist. Der Luftstrom wird beim Einatmen durch das Mundrohr so geführt, daß die freigesetzte Pulvermenge mit der Atemluft vermischt wird.

Für die Ausgestaltung des Inhalators bestehen zahlreiche Möglichkeiten. Beispielsweise kann zur verbesserten Koordination der Pulverfreisetzung mit dem Einatmen ein Mechanismus eingebaut werden, mit dessen Hilfe durch den Atemzug die Sperre gelöst wird, welche die Feder 7 im gespannten Zustand hält.

Es ist auch möglich, das Inhalationspulver zunächst in ein größeres Gefäß mit Mundrohr und Lufteinlaß (z.B. gemäß dem deutschen Gebrauchsmuster G 8908273 oder gemäß EP-A-9667) zu blasen und dann durch dieses Gerät zu inhalieren. Auf diese Weise können Freisetzung des Inhalationspulvers und Inhalationsvorgang zeitlich noch stärker voneinander getrennt werden.

Die Anordnung von Sektoren mit Wirkstoff auf einer Trägerscheibe ist Fig. 2 zu entnehmen. Die einzelnen Sektoren, von denen jeder eine Wirkstoffdosis enthält, sind in diesem Fall durch Zwischenwände voneinander abgegrenzt. Ein (hier nicht dargestellter) Mechanismus bewirkt, daß die Scheibe bei jeder Betätigung des Geräts um einen Sektor weitergedreht wird. Die in Fig. 2a erkennbaren Sektoren (Felder) weisen erfindungsgemäße Mikrostrukturen auf (nicht eingezeichnet). Fig. 2b zeigt - vergrößert - die Anordnung von Noppen auf einem solchen Feld. Auch hier vergrößert sich der Abstand der Noppen in einem Ring voneinander nach außen hin entsprechend dem größeren Umfang des Ringes, wobei der Abstand von Ring zu Ring im allgemeinen konstant ist. Die Felder mit jeweils einer Wirkstoffdosis sind durch Zwischenwände 11 voneinander getrennt.

Statt durch Zwischenwände können die einzelnen Pulverdosen auch durch nichtgefüllte Flächen voneinander getrennt sein oder durch eine Maske geführt werden, die jeweils nur eine solche Fläche dem Luftstoß aus der Düse 10 aussetzt, die mit genau einer Pulverdosis beladen ist. Eine bevorzugte Ausführungsform dieser Art enthält die Trägerscheibe in einer Kassette, so daß alle anderen Sektoren gegen Berührung geschützt sind. Der Ausschnitt, in dem bei der Benutzung die Pulverdosis freigelegt werden soll, kann auch durch einen Verschlußschieber oder eine entsprechende Vorrichtung derart verschlossen sein, daß die Öffnung erst beim Einlegen der Trägerscheibe in den Inhalator freigegeben wird.

Es hat sich gezeigt, daß auch ein gleichmäßig beladener Träger benutzt werden kann, von dem nur ein Teil des Medikaments abgeblasen wird, weil die von einem solchen Träger abgeblasene Dosis eine gute Konstanz zeigt.

Die Drehung der Trägerscheibe um einen Sektor kann z.B. mit dem Spannen der Feder 7 so gekoppelt sein, daß bei jedem Spannen die Trägerscheibe um einen Sektor weitergedreht wird. Die Trägerscheibe kann mit Einrichtungen versehen sein, die bewirken, daß die Sektoren jeweils in der richtigen Position zur Düse stehen, etwa durch Kerben in entsprechenden Abständen und einrastende gefederte Elemente.

## Patentansprüche

1. Pulverinhalator zur Inhalation von therapeutisch wirksamen Pulvern von der Oberfläche von flachen Trägern (1), wobei der Inhalator einen flachen Träger (1) mit regelmäßig angeordneten Mikrostrukturen (3) aufweist und mit einer Vorrichtung (4,5,6,7,8,9,10) zur Erzeugung eines Luftstoßes versehen ist, mit dessen Hilfe jeweils eine Dosis des Pulvers zwischen den regelmäßig angeordneten Mikrostrukturen (3) eines Trägers (1) herausgeblasen und in ein Aerosol überführt wird, das unmittelbar oder nach Speicherung in einem zwischengeschalteten Gefäß der Atemluft eines Patienten zugemischt wird, dadurch gekennzeichnet, daß die Mikrostrukturen (3) noppenartige Erhebungen, die über die das Pulver tragende Oberfläche des Trägers heraussfehen, sind und eine Höhe im Bereich von 10 bis 500 µm aufweisen,wobei es sich bei besagten Mikrostrukturen (3) nicht um samt-oder veloursartig angeordnete Fasern handelt.

2. Pulverinhalator nach Anspruch 1, dadurch gekennzeichnet, daß die Mikrostrukturen (3) eine Höhe im Bereich von 50 bis 200 µm aufweisen.

3. Pulverinhalator nach Anspruch 1 oder 2, dadurch gekennzeichnet, daß der Träger (1) band- oder platten- bzw. scheibenförmig ist.

4. Pulverinhalator nach einem der Ansprüche 1 bis 3, dadurch gekennzeichnet, daß die Mikrostrukturen (3) einen kreisförmigen, elliptischen, vieleckigen, Y- oder kreuzförmigen oder einen aus solchen Formen kombinierten Querschnitt haben.

5. Pulverinhalator nach einem der Ansprüche 1 - 4, dadurch gekennzeichnet, daß die auf die Trägerebene gerichtete Achsen der Mikrostrukturen (3) auf dieser senkrecht stehen oder auch mit ihr einen Winkel <90° bilden.

6. Pulverinhalator nach einem der Ansprüche 1 - 5, dadurch gekennzeichnet, daß die Mikrostrukturen (3) zylindrisch, prismatisch, kegel- bzw. kegelstumpfförmig, oder pyramiden- bzw. pyramidenstumpfförmig sind.

7. Pulverinhalator nach einem der Ansprüche 1 - 6, dadurch gekennzeichnet, daß der Durchmesser bzw. die entsprechenden Abmessungen der Mikrostrukturen (3) bei nichtkreisförmigem Querschnitt zwischen etwa 10 und 500 µm, vorzugsweise zwischen 30 und 100 µm betragen.

8. Pulverinhalator nach einem der Ansprüche 1 bis 7, dadurch gekennzeichnet, daß der Abstand zwischen den Mikrostrukturen (3) etwa 10 und 500 µm, vorzugsweise zwischen 30 und 100 µm beträgt.

9. Pulverinhalator nach einem der Ansprüche 1 bis 8, dadurch gekennzeichnet, daß das Verhältnis von freiem Volumen zwischen den Mikrostrukturen (3) zum gesamten Raum, der mit Mikrostrukturen ausgefüllt ist, 10 bis 98 %, vorzugsweise 50 bis 80 % beträgt.

10. Pulverinhalator nach einem der Ansprüche 1 bis 9 dadurch gekennzeichnet, daß der Träger (1) aus biegsamem oder starrem, Material besteht.

11. Pulverinhalator nach Anspruch 10 dadurch gekennzeichnet, daß der Träger (1) die Form einer starren bzw. steifen Kreisscheibe aufweist, auf der die einzelnen Dosen des Inhalationspulvers in Sektoren angeordnet sind, wobei die einzelnen Sektoren gewünschtenfalls durch Zwischenwände voneinander getrennt sind.

## Claims

1. Powder inhaler for the inhalation of therapeutically active powders from the surface of flat carriers (1) wherein the inhaler comprises a flat carrier (1) with regularly arranged microstructures (3) and with a device (4,5,6,7,8,9,10) for generating a blast of air by means of which a dose of the powder between the regularly arranged microstructures (3) of a carrier (1) is blown out and converted into an aerosol which is mixed with the patient's breath immediately or after storage in an intermediate container, characterised in that the microstructures (3) are knob-like elevations which project above the surface of the carrier holding the powder and have a height in the range from 10 to 500 µm, said microstructures (3) not being fibres arranged in the manner of velvet or velours.

2. Powder inhaler according to claim 1, characterised in that the height of the microstructures (3) is in the range from 50 to 200 µm.

3. Powder inhaler according to claim 1 or 2, characterised in that the carrier (1) is in the form of a belt, plate or disc.

4. Powder inhaler according to one of claims 1 to 3, characterised in that the microstructures (3) are circular, elliptical, polygonal, Y-shaped or cross-shaped in cross-section or have a cross-section which is a combination of such shapes.

5. Powder inhaler according to one of claims 1 to 4, characterised in that the axes of the microstructures (3) directed towards the plane of the carrier are perpendicular thereto or form an angle of < 90° therewith.

6. Powder inhaler according to one of claims 1 to 5, characterised in that the microstructures (3) are cylindrical, prismatic, conical or frustoconical, or in the form of pyramids or truncated pyramids.

7. Powder inhaler according to one of claims 1 to 6, characterised in that the diameter or the corresponding dimensions of the microstructures, in the case of a non-circular cross-section, are between about 10 and 500 µm, preferably between 30 and 100 µm.

8. Powder inhaler according to one of claims 1 to 7, characterised in that the spacing between the microstructures (3) is between about 10 and 500 µm, preferably between 30 and 100 µm.

9. Powder inhaler according to one of claims 1 to 8, characterised in that the ratio of free volume between the microstructures (3) to the total space filled with microstructures is from 10 to 98%, preferably from 50 to 80%.

10. Powder inhaler according to one of claims 1 to 9, characterised in that the carrier (1) consists of flexible or rigid material.

11. Powder inhaler according to claim 10, characterised in that the carrier (1) takes the form of a rigid or stiff circular disc on which the individual doses of the powder for inhalation are arranged in sectors, the individual sectors being separated from one another, if desired, by means of partition walls.

## Revendications

1. Inhalateur de poudre pour l'inhalation de poudres thérapeutiquement actives de la surface de supports plats (1), où l'inhalateur présente un support plat (1) avec des micro-structures régulièrement disposées (3) et est muni d'un dispositif (4,5,6,7,8,9,10) pour la production d'un courant d'air à l'aide duquel une dose de la poudre est soufflée au dehors dans chaque cas entre les microstructures régulièrement disposées (3) d'un support (1) et est transférée dans un aérosol, qui est ajouté à l'air inhalé d'un patient immédiatement ou après stockage dans un récipient intercalé, caractérisé en ce que les micro-structures (3) sont des protubérances de type nopes, qui font saillie au-dessus de la surface du support portant la poudre et présentent une hauteur dans le domaine de 10 à 500 µm, où, concernant lesdites micro-structures (3), il ne s'agit pas de fibres disposées à la manière du velours.

2. Inhalateur de poudre selon la revendication 1, caractérisé en ce que les micro-structures (3) présentent une hauteur dans le domaine de 50 à 200 µm.

3. Inhalateur de poudre selon la revendication 1 ou 2, caractérisé en ce, que le support (1) est en forme de bande ou de plaque ou disque.

4. inhalateur de poudre selon l'une des revendications 1 à 3, caractérisé en ce que les micro-structures (3) ont une section droite circulaire, elliptique, polygonale, en forme de Y ou de croix ou une section droite combinée à partir de telles formes.

5. Inhalateur de poudre selon l'une des revendications 1 à 4, caractérisé en ce que les axes des micro-structures (3) dirigés vers le plan du support sont situés verticalement par rapport à lui ou bien forment avec lui un angle <90°.

6. Inhalateur de poudre selon l'une des revendications 1 à 5, caractérisé en ce que les micro-structures (3) sont cylindriques, prismatiques, coniques ou tronconiques, ou pyramidales ou pyramidales tronquées.

7. Inhalateur de poudre selon l'une des revendications 1 à 6, caractérisé en ce que le diamètre ou les dimensions correspondantes des micro-structures (3) pour une section droite non circulaire sont d'environ 10 à 500 µm, de préférence de 30 à 100 µm.

8. Inhalateur de poudre selon l'une des revendications 1 à 7, caractérisé en ce que la distance entre les micro-structures (3) est d'environ 10 à 500 µm, de préférence de 30 à 100 µm.

9. Inhalateur de poudre selon l'une des revendications 1 à 8, caractérisé en ce que le rapport du volume libre entre les micro-structures (3) à l'espace total qui est rempli par les micro-structures est de 10 à 98%, de préférence de 50 à 80%.

10. Inhalateur de poudre selon l'une des revendications 1 à 9 caractérisé que le support (1) consiste en matériau pliable ou rigide.

11. Inhalateur de poudre selon la revendication 10 caractérisé que le support (1) présente la forme d'une plaque circulaire rigide ou inflexible sur laquelle les doses individuelles de la poudre d'inhalation sont disposées en secteurs, les secteurs individuels étant si on le souhaite séparés les uns des autres par des cloisons.
